(19) Europäisches Patentamt
European Patent Office
Office européen des brevets

(11) Veröffentlichungsnummer: **0 045 425**
**B1**

# (12) EUROPÄISCHE PATENTSCHRIFT

(45) Veröffentlichungstag der Patentschrift: **23.11.83**

(51) Int. Cl.³: **C 07 C 17/14**, C 07 B 9/00, C 07 C 21/24

(21) Anmeldenummer: **81105674.6**

(22) Anmeldetag: **20.07.81**

(54) **Verfahren zur Halogenierung von gegebenenfalls substituierten 4-tert.-Butyltoluolen sowie die daraus erhaltenen Gemische aus gegebenenfalls substituierten 4-tert.-Butylbenzalhalogeniden, 4-tert.-Butylbenzylhalogeniden und 4-tert.-Butylbenzotrihalogeniden.**

(30) Priorität: **01.08.80 DE 3029368**

(43) Veröffentlichungstag der Anmeldung: **10.02.82 Patentblatt 82/6**

(45) Bekanntmachung des Hinweises auf die Patenterteilung: **23.11.83 Patentblatt 83/47**

(84) Benannte Vertragsstaaten: **BE CH DE FR GB IT LI**

(56) Entgegenhaltungen:
**EP - A - 0 011 281**
**EP - A - 0 011 288**

**Chemical Abstracts Band 54, Nr. 15, 1960 Columbus, Ohio, USA R.E. BUCKLES et al. "Addition reactions of mixtures of bromine and chlorine" Spalte 15304b-h**
**JOURNAL OF ORGANIC CHEMISTRY, Band 43, Nr. 14, 1978 Columbus G.E. HEASLEY et al. "Electrophilic Additions to Dienes and the 1-Phenyl-propenes with Pyridine-Halogen Complexes and Tribomides. Effects on Stereochemistry and Product Ratios" Seiten 2793 bis 2799**
**Patents Abstracts of Japan Band 3, Nr. 72, 21. Juni 1979 Seite 18C49**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 045 425 B1

ACTORUM AG

# Verfahren zur Halogenierung von gegebenenfalls substituierten 4-tert.-Butyltoluolen sowie die daraus erhaltenen Gemische aus gegebenenfalls substituierten 4-tert.-Butylbenzalhalogeniden, 4-tert.-Butylbenzylhalogeniden und 4-tert.-Butylbenzotrihalogeniden

Die Erfindung betrifft ein Verfahren zur Halogenierung von gegebenenfalls substituierten 4-tert.-Butyltoluolen sowie die daraus erhaltenen Gemische aus gegebenenfalls substituierten 4-tert.-Butylbenzalhalogeniden, 4-tert.-Butylbenzylhalogeniden und 4-tert.-Butylbenzotrihalogeniden.

Aus der DE-OS 2 849 692 ist u.a. bekannt, 4-tert.-Butylbenzalbromid und dessen am Kern durch Halogen substituierte Derivate dadurch herzustellen, dass man 4-tert.-Butyltoluol und dessen am Kern durch Halogen substituierte Derivate mit etwa 2 Mol Brom pro Mol organisches Ausgangsmaterial bei Temperaturen von 40 bis 200°C gegebenenfalls unter Einwirkung energiereicher Strahlung oder in Gegenwart von Radikalbildnern umsetzt.

In der DE-OS 2 849 663 ist ein analoges Verfahren zur Herstellung von 4-tert.-Butylbenzotribromiden beschrieben.

Auf Seite 4 der erwähnten Offenlegungsschriften wird ausgeführt, dass die radikalische Chlorierung von 4-tert.-Butyltoluol nicht nur das erwünschte 4-tert.-Butylbenzotrichlorid bzw. das 4-tert.-Butylbenzalchlorid ergibt, sondern dass dabei in beträchtlichem Masse kernchlorierte Produkte erhalten werden, was aus dem Befund gefolgert wird, dass das eingeführte Chlor nur z. T. durch alkalische Hydrolyse wieder abgespalten werden kann.

Ausserdem zeigen eigene Versuche, dass in erheblichem Masse Chlor in die tert.-Butylgruppe eintritt, was ebenfalls zu unerwünschten Nebenprodukten führt.

Von Nachteil ist bei den in den erwähnten Offenlegungsschriften beschriebenen Verfahren, dass das für die Bromierung verwendete Brom relativ teuer ist, was die Wirtschaftlichkeit der Verfahren beeinträchtigt.

Es wurde nun ein Verfahren zur Halogenierung von gegebenenfalls substituiertem 4-tert.-Butyltoluol gefunden, das dadurch gekennzeichnet ist, dass man gegebenenfalls substituiertes 4-tert.-Butyltoluol mit 0,5 bis 3,2 Mol Bromchlorid, gegebenenfalls in Gegenwart von Chlor und/oder Brom, als Halogenierungsmittel, pro Mol gegebenenfalls substituiertes 4-tert.-Butyltoluol, bei Temperaturen von 40 bis 230°C, gegebenenfalls in Gegenwart von inerten organischen Lösungs- oder Verdünnungsmitteln, umsetzt.

Ausgangsprodukte für das erfindungsgemässe Verfahren sind 4.-tert.-Butyltoluol sowie dessen am Kern ein- oder mehrfach durch Halogenatome, wie Fluor, Chlor, Brom oder Jod substituierte Derivate, wobei dann natürlich am Ende der Reaktion jeweils die gleiche Substitution des Kerns im Endprodukt vorhanden ist.

Die monosubstituierten Derivate können durch folgende allgemeine Formel (I) wiedergegeben werden:

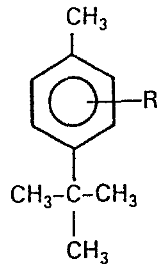

(I),

worin

R für Fluor, Chlor, Brom oder Jod steht.

Bevorzugt wird 4.-tert.-Butyltoluol in das erfindungsgemässe Verfahren eingesetzt.

Die Zusammensetzung der erfindungsgemäss erhaltenen Gemische aus gegebenenfalls substituierten 4.-tert.-Butylbenzalhalogeniden, 4.-tert.-Butylbenzylhalogeniden und 4.-tert.-Butylbenzotrihalogeniden kann durch die jeweils eingesetzte Menge an Halogenierungsmittel variiert werden.

So kann nach dem erfindungsgemässen Verfahren bei vollständigem Umsatz des eingesetzten Produkts durch Einsatz von 1,9 bis 2,2, vorzugsweise 2,0 bis 2,1 Mol Halogenierungsmittel pro Mol eingesetztes Produkt ein Halogenierungsgemisch erhalten werden, das gegebenenfalls substituiertes 4.-tert.-Butylbenzalhalogenid in einer Menge von 90 bis 93 Gew.-% enthält. 4.-tert.-Butylbenzylhalogenid und 4.-tert.-Butylbenzotrihalogenid, die ebenfalls substituiert sein können, werden dabei nur in untergeordneten Mengen von 3 bis 4 Gew.-% bzw. 4 bis 6 Gew.-%, gebildet.

Bei Einsatz von 0,5 bis 1,9, bevorzugt 1,2 bis 1,8, besonders bevorzugt 1,5 bis 1,7 Mol Halogenierungsmittel pro Mol Ausgangsprodukt ist es möglich, Halogenierungsgemische herzustellen, in denen Benzotrihalogenide unter 1 Gew.-%, vorzugsweise unter 0,1 Gew.-%, enthalten sind.

Bei vollständigem Umsatz des Ausgangsproduktes mit 1,2 bis 1,8, bevorzugt 1,5 bis 1,7 Mol Halogenierungsmittel pro Mol Ausgangsprodukt wird beispielsweise ein Halogenierungsgemisch erhalten, das 40 bis 85 Gew.-%, bevorzugt 80 bis 85 Gew.-%, gegebenenfalls substituiertes 4.-tert.-Butylbenzalhalogenid neben 15 bis 60 Gew.-%, bevorzugt 15 bis 20 Gew.-%, gegebenenfalls substituiertem 4.-tert.-Butylbenzylhalogenid enthält.

Zur Erhöhung der Ausbeute an gegebenenfalls substituiertem 4-tert.-Butylbenzalhalogenid kann man einen Teil des Halogenierungsgemisches, etwa entsprechend dem Anteil an gegebenenfalls substituiertem 4-tert.-Butylbenzylhalogenid, durch z.B. Destillation abtrennen und zur weiteren Halogenierung wieder in das Reaktionsgemisch zurückführen.

Durch diese Verfahrensvariante gelingt es, die Gehalte an gegebenenfalls substituiertem 4-tert.-Butylbenzalhalogenid auf 95 bis 100 Gew.-% zu steigern. Daneben kann das Gemisch noch 0 bis 5 Gew.-%, vorzugsweise weniger als 1 Gew.-%, insbesondere weniger als 0,1 Gew.-% an gegebenenfalls substituiertem 4-tert.-Butylbenzylhalogenid enthalten.

Weiterhin ist es möglich, 1,0 Mol gegebenenfalls substituiertes 4-tert.Butyltoluol vollständig mit 2,2 bis 3,2 Mol, vorzugsweise 2,3 bis 2,7 Mol, insbesondere 2,4 bis 2,5 Mol Halogenierungsmittel umzusetzen. Dabei werden Halogenierungsgemische erhalten, die 10 bis 95 Gew.-%, vorzugsweise 20 bis 80 Gew.-%, insbesondere 30 bis 60 Gew.-% an gegebenenfalls substituiertem 4-tert.-Butylbenzotrihalogenid und 5 bis 90 Gew.-%, vorzugsweise 20 bis 80 Gew.-%, insbesondere 40 bis 70 Gew.-% an gegebenenfalls substituiertem 4-tert.-Butylbenzalhalogenid enthalten.

Das in den Halogenierungsgemischen enthaltene 4-tert.-Butylbenzalhalogenid enthält 5 bis 30 Gew.-%, vorzugsweise 10 bis 25 Gew.-%, insbesondere 20 Gew.-% eines Benzalhalogenids der Formel (II)

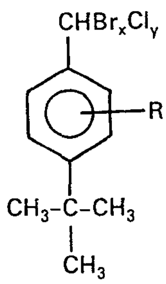

(II),

worin
X = 0 oder 1 und Y = 1 oder 2 sein kann, wobei die Summe von X + Y = 2 ist, bevorzugt
X = 1 und Y = 1 ist, und
R Wasserstoff, Fluor, Chlor, Brom oder Jod bedeutet.

Der Rest besteht aus gegebenenfalls substituiertem 4-tert.-Butylbenzalbromid.

Das in den Halogenierungsgemischen enthaltene, gegebenenfalls substituierte 4-tert.-Butylbenzylhalogenid enthält überwiegend (70–95 Gew.-%) die entsprechende Bromverbindung und zu einem geringeren Anteil (5–30 Gew.-%) die entsprechende Chlorverbindung.

Das in den Halogenierungsgemischen enthaltene 4-tert.-Butylbenzotrihalogenid enthält in untergeordnetem Masse (5–30 Gew.-%) 4-tert.-Butylbenzotrihalogenid der Formel (III)

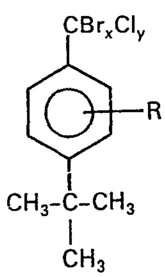

(III),

worin
X = 1 oder 2 und Y = 1 oder 2 sein kann, wobei die Summe X + Y = 3 ist, und
R Wasserstoff, Fluor, Chlor, Brom oder Jod bedeutet.

Im überwiegenden Masse besteht das 4-tert.-Butylbenzotrihalogenid aus der entsprechenden Bromverbindung (70–95 Gew.-%).

Das erfindungsgemässe Verfahren wird bevorzugt bei Temperaturen von 100 bis 200°C, besonders bevorzugt bei 160 bis 190°C, durchgeführt.

Als organische Lösungs- oder Verdünnungsmittel können solche in das erfindungsgemässe Verfahren eingesetzt werden, die sich unter den Reaktionsbedingungen inert verhalten.

Die organischen Lösungs- oder Verdünnungsmittel können sowohl einzeln als auch im Gemisch untereinander eingesetzt werden.

Zum Beispiel können halogenierte aliphatische Kohlenwasserstoffe mit 1 bis 5 Kohlenstoffatomen, bevorzugt 1 bis 2 Kohlenstoffatomen, wie Dichlormethan, Trichlormethan, Tetrachlormethan, Dichlorethan, Trichlorethan, Tetrachlorethan und Hexachlorethan, bevorzugt Tetrachlormethan, sowie gegebenenfalls halogenierte aromatische Kohlenwasserstoffe, wie Benzol, Brombenzol, Chlorbenzol, Dichlorbenzol, bevorzugt Dichlorbenzol, in das erfindungsgemässe Verfahren eingesetzt werden.

Die optimale Menge an einzusetzenden Lösungs- oder Verdünnungsmitteln hängt u.a. von der Reaktionstemperatur ab und kann leicht durch Vorversuche ermittelt werden.

Im allgemeinen werden die inerten organischen Lösungs- oder Verdünnungsmittel in Mengen von bis zu 1000 Vol.-%, bevorzugt 5 bis 50 Vol.-%, bezogen auf das eingesetzte Produkt, eingesetzt.

Vorzugsweise wird ohne Lösungs- oder Verdünnungsmittel gearbeitet.

Ein besonderes Merkmal des erfindungsgemässen Verfahrens ist es, dass die Umsetzung ohne Verwendung von Radikalbildnern, Katalysatoren oder sonstigen Hilfsstoffen und ohne die Mitwirkung von natürlichem oder künstlichem Licht durchgeführt werden kann.

Selbstverständlich kann das erfindungsgemässe Verfahren auch unter Verwendung von Radikalbildnern, Katalysatoren oder sonstigen Hilfsstoffen und unter Einwirkung von natürlichem oder künstlichem Licht durchgeführt werden. Allerdings bringt diese Verfahrensweise keine besonderen Vorteile.

Bei Verwendung von organischen Lösungs- oder Verdünnungsmitteln kann es auch vorteilhaft sein, in Gegenwart von Wasser in einem Zweiphasensystem zu arbeiten, wobei die Wasserphase z.B. durch Zusatz von Alkali- oder Erdalkaliverbindungen, wie den Oxiden, Hydroxiden, Carbonaten, Hydrogencarbonaten von Lithium, Natrium, Kalium, Magnesium, Calcium, sowie den entsprechenden Aluminiumverbindungen teilweise oder vollständig neutralisiert wird (etwa pH 3 bis 9, vorzugsweise pH 6 bis 7, insbesondere etwa pH 7). Diese Variante wird bevorzugt bei etwa 40 bis 80°C unter Rückflussbedingungen durchgeführt.

In das erfindungsgemässe Verfahren kann sowohl reines Ausgangsprodukt als auch technisches Ausgangsprodukt mit einem Gehalt von ca. 95 bis 98 Gew.-% an gegebenenfalls substituiertem 4-tert.-Butyltoluol neben ca. 2 bis 3 Gew.-% isomeren tert.-Butyltoluolen, wie 3-tert.-Butyltoluol, geringen Mengen gegebenenfalls substituiertem Toluol sowie Spuren verschiedener anderer Verbindungen, wie Oligomere des Isobutens eingesetzt werden.

Es ist auch möglich, stärker verunreinigtes Ausgangsprodukt, das etwa 1 bis 5 Gew.-% Oligomere des Isobutens neben diversen anderen Verbindungen wie Spuren von Schwefelsäure, Di-tert.-butylether, Mono- und Di-tert.-butylsulfat enthält, in das erfindungsgemässe Verfahren einzusetzen. So ist es z.B. möglich, ein aus der Alkylierung von Toluol stammendes undestilliertes 4-tert.-Butyltoluol, das gegebenenfalls von überschüssigem Toluol befreit wurde, in das erfindungsgemässe Verfahren einzusetzen.

Im allgemeinen wird das erfindungsgemässe Verfahren so durchgeführt, dass man technisches gegebenenfalls substituiertes 4-tert.-Butyltoluol mit einem Gehalt von ca. 95 bis 97 Gew.-% an gegebenenfalls substituiertem 4-tert.-Butyltoluol, gegebenenfalls mit einem inerten Lösungs- oder Verdünnungsmittel, auf etwa 160 bis 190°C aufheizt und bei guter Durchmischung die berechnete Menge an Halogenierungsmittel gasförmig oder flüssig in der Weise eindosiert, dass es unterhalb der Oberfläche des Reaktionsgemisches, zweckmässigerweise in der unteren Hälfte des Reaktionsgemisches, z.B. in der Nähe des Bodens des Reaktionsgefässes, eintritt. Dies hat den Vorteil, dass praktisch kein Halogenierungsmittel aus dem Reaktionsgemisch mehr entweicht.

Das als Halogenierungsmittel verwendete Bromchlorid, auch als Chlorbrom oder Bromchlor bekannt, kann in Gegenwart von Brom und/oder Chlor verwendet werden. Das Halogenierungsmittel wird beispielsweise in flüssiger Form oder in gelöster Form in üblicher Weise über eine Dosiervorrichtung in das Reaktionsgemisch eingebracht, wobei darauf geachtet wird, dass die Dosierung in dem Masse erfolgt, wie das Halogenierungsmittel im Ansatz verbraucht wird, so dass gewöhnlich bei einer stationären Konzentration an Halogenierungsmittel von unter 5 Gew.-%, vorzugsweise unter 2 Gew.-% und insbesondere unter 1 Gew.-% gearbeitet wird. Ebenso ist es möglich, das Halogenierungsmittel vor dem Reaktor zu verdampfen und gasförmig in den Reaktor einzudosieren, wobei gegebenenfalls in Gegenwart eines Inertgases gearbeitet wird. Als Inertgase können beispielsweise Stickstoff, Chlorwasserstoff, Bromwasserstoff, Kohlendioxid und Schwefeldioxid verwendet werden. Vorzugsweise verwendet man Stickstoff.

Das Halogenierungsmittel wird gewöhnlich durch Vermischen von Brom mit Chlor und/oder durch Vermischen von Bromwasserstoff mit Chlor hergestellt. Beispielsweise vermischt man Brom und/oder Bromwasserstoff mit Chlor im Verhältnis 10 Brom:1 Chlor bis 1 Brom:10 Chlor. Vorzugsweise enthält das Bromierungsmittel Brom und Chlor im Verhältnis 2:1 bis 1:2 und insbesondere im Verhältnis von etwa 1:1. Das Halogenierungsmittel kann in der Flüssig- oder Gasphase hergestellt werden, gegebenenfalls in Gegenwart eines gasförmigen oder flüssigen Verdünnungsmittels. Ein entsprechend hergestelltes gasförmiges bromchlorhaltiges Halogenierungsmittel kann direkt für das erfindungsgemässe Verfahren verwendet werden. Ebenso ist es möglich, ein solches Halogenierungsmittel durch Kühlung zu kondensieren oder in einem Lösungsmittel zu lösen. Ebenso können entsprechend hergestellte Bromchlor enthaltende Halogenierungsmittel, die in der Flüssigphase, gegebenenfalls in Gegenwart eines Lösungsmittels, hergestellt wurden, direkt für das erfindungsgemässe Verfahren verwendet werden.

Bei rascher Halogenierungsmittelzugabe ist es vorteilhaft, das unverbrauchte Halogenierungsmittel zurückzugewinnen oder in das Reaktionsgemisch zurückzuführen, wodurch das Halogenierungsmittel durch Umsetzung mit dem Ausgangsprodukt aus dem Abgasstrom teilweise oder vollständig entfernt wird.

Es ist auch möglich, das gegebenenfalls substituierte 4-tert.-Butyltoluol simultan mit dem Halogenierungsmittel in den Reaktor einzudosieren, wobei die simultane Zugabe sowohl chargenweise als auch kontinuierlich durchgeführt werden kann.

Nachdem die berechnete Menge an Halogenierungsmittel zugegeben worden ist, wird bis zum Ende der Halogenwasserstoffentwicklung nachgerührt.

Die vorherige Aufarbeitung des Reaktionsgemisches ist bei lösungsmittelfreier Arbeitsweise gewöhnlich nicht erforderlich, wenn das Gemisch weiterverarbeitet werden soll. Ein Halogenierungsgemisch, das beispielsweise 90 bis 93 Gew.-% gegebenenfalls substituiertes 4-tert.-Butylbenzalhalogenid enthält, kann in dieser Form, gegebenenfalls nach Entfernung von noch gelöstem Halogenwasserstoff im Vakuum, direkt weiterverarbeitet werden. Bei Bedarf kann eine weitere Reinigung oder gegebenenfalls Fraktionierung des Reaktionsgemisches durch Vakuumdestillation oder Umkristallisation erfolgen.

Das erfindungsgemässe Verfahren kann sowohl kontinuierlich als auch diskontinuierlich durchgeführt werden.

Nach dem erfindungsgemässen Verfahren ist es insbesondere möglich, gegebenenfalls substituierte 4-tert.-Butylbenzalhalogenide in guten Ausbeuten und mit einer hohen Selektivität herzustellen.

Dass es nach dem erfindungsgemässen Verfahren gelingt, gegebenenfalls substituierte 4-tert.-Butylbenzalhalogenide in guten Ausbeuten zu erhalten, überrascht ausserordentlich, da, wie eingangs geschildert, erwartet werden musste, dass mit z.B. Bromchlorid neben der gewünschten Seitenkettenhalogenierung auch die eingangs geschilderten Nebenreaktionen stattfinden würden, was natürlich zu Lasten der Ausbeute an 4-tert.-Butylbenzalhalogeniden ginge.

Die nach dem erfindungsgemässen Verfahren herstellbaren Halogenierungsgemische, deren Zusammensetzung, wie zuvor beschrieben, durch die Variation der zugegebenen Halogenierungsmittelmenge geändert werden kann, werden gewöhnlich zur Herstellung von Benzaldehyden der allgemeinen Formel

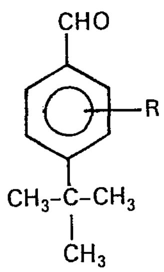

(IV),

worin
R für Wasserstoff, Fluor, Chlor, Brom oder Jod steht,
oder auch zur Herstellung von Säurederivaten bzw. von Säuren der allgemeinen Formel (V)

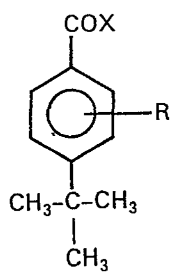

(V),

worin
X Chlor, Brom oder Hydroxy bedeutet und
R für Wasserstoff, Fluor, Chlor, Brom oder Jod steht,
verwendet.

Zum Beispiel dient der 4-tert.-Butylbenzaldehyd als Ausgangsprodukt für Riechstoffe (s. Riechstoff Ind. 13, 81 [1938]) und die 4-tert.-Butylbenzoesäure als Komponente für Lackrohstoffe und Alkydharze (s. Römpp, 7. Auflage, Band 1, Seite 455).

Die nachfolgenden Beispiele sollen das erfindungsgemässe Verfahren verdeutlichen,

Beispiel 1

In einem 500 ml Dreihalskolben mit Tropftrichter, dessen Eintropfrohr bis an den Boden des Kolbens reicht, werden 120,0 g 4-tert.-Butyltoluol (97 %ig, 2 % Isomeres; 0,80 Mol) unter Rühren auf 160 bis 170°C aufgeheizt und unter Lichtausschluss im Verlauf von 6 Stunden 196,4 g Bromchlorid (1,7 Mol) so zugetropft, dass im Rückflusskühler kein Bromchlorid erscheint. Es wird noch ca. 10 Minuten bis zum Ende der Gasentwicklung nachgerührt.

Das Reaktionsgemisch erstarrt bei Raumtemperatur zu 237 g eines hellbraunen Kristallkuchens, der ca. 90 % 4-tert.-Butylbenzalhalogenid, und jeweils ca. 3 % Benzylhalogenid und Benzotrihalogenid enthält. Die Ausbeute an Benzalhalogenid beträgt ca. 90 %, bezogen auf eingesetztes Toluol.

Beispiel 2

Apparatur und Ansatz wie in Beispiel 1 beschrieben. Dem Ansatz werden 75 ml (119 g) Tetrachlormethan hinzugefügt. Verdampfendes Tetrachlormethan wird kondensiert und zusammen mit Bromchlorid dem Reaktionsgemisch wie in Beispiel 1 geschildert, zugeführt. Die Temperatur wird zwischen 110 und 120°C gehalten. Nach 8 Stunden ist alles Brom aufgenommen. Nachdem Tetrachlormethan abdestilliert ist, werden 223 g eines Gemisches erhalten, das 15,5 % Benzylhalogenide, 73,3 % Benzalhalogenide und 11,0 % Benzotrihalogenide enthält. Die Ausbeute an Benzalhalogenid beträgt ca. 71 %, bezogen auf eingesetztes Toluol.

Beispiel 3

Apparatur und Ansatz wie in Beispiel 2 beschrieben.

Statt 75 ml werden 40 ml (64 g) Tetrachlorkohlenstoff hinzugefügt. Die Temperatur wird zwischen 160 und 170°C gehalten. Nach 2,5 Stunden ist alles Bromchlorid umgesetzt. Nachdem das Verdünnungsmittel abdestilliert ist, werden 226 g eines Gemisches mit 6,8 % Benzylhalogenid, 82,3 % Benzalhalogenid und 9,9 % Benzotrihalogenid erhalten. Dies entspricht einer Ausbeute an Benzalhalogenid von 80 %, bezogen auf eingesetztes Toluol.

Beispiel 4

Apparatur und Ansatz wie in Beispiel 1 beschrieben.

Dem Ansatz werden 300 ml (389 g) Dichlorbenzol hinzugefügt. Nach 3 Stunden bei 160 bis 170°C ist das Bromchlorid umgesetzt. Nach dem Abdestillieren des Dichlorbenzols werden 230 g eines Gemisches erhalten, das 2,3 % Benzylhalogenid, 89,4 % Benzalhalogenid und 7,9 % Benzotrihalogenid enthält. Dies entspricht einer Ausbeute an Benzalhalogenid von 89 %, bezogen auf eingesetztes Toluol.

Beispiel 5

Apparatur und Ansatz wie in Beispiel 1 beschrieben. Es wird eine Temperatur von 190°C eingehalten. Nach 4 Stunden ist alles Bromchlorid eindosiert und umgesetzt. Man erhält 234 g eines Gemisches mit 1,8 % Benzylhalogenid, 90,2 % Benzalhalogenid und 7,1 % Benzotrihalogenid. Dies entspricht einer Ausbeute an Benzalhalogenid von 91 %, bezogen auf eingesetztes Toluol.

Beispiel 6

Apparatur und Ansatz wie in Beispiel 1 beschrieben.

Es wird eine Temperatur von 220 bis 230°C gehalten. Im ersten Drittel der Reaktion steigt die Temperatur von 190 auf 220°C (der Siedepunkt des 4-tert.-Butyltoluols beträgt 193°C bei Normaldruck). Nach 1 Stunde und 15 Minuten ist alles Bromchlorid aufgenommen. Man erhält 225 g eines Gemisches, das 9,7 % Benzylhalogend, 82,1 % Benzalhalogenid, 1,6 % Benzotrihalogenid und 6,4 % höher siedende Kondensationsprodukte enthält. Die Ausbeute an Benzalhalogenid entspricht 80 %, bezogen auf eingesetztes Toluol.

Beispiel 7

Apparatur und Ansatz wie in Beispiel 1 beschrieben.

Es werden jedoch nur 174,0 g (1,51 Mol) Bromchlorid eingesetzt. Nach 4 Stunden ist alles Bromchlorid eindosiert und umgesetzt. Man erhält 224 g eines Gemisches, das 17,7 % Benzylhalogenid, 82,5 % Benzalhalogenid und 0,4 % Benzotrihalogenid enthält. Über eine Kolonne werden bei einem Druck von 1 mbar 70 g des Reaktionsgemisches bis zu einer Kopftemperatur von 130°C abdestilliert. Es bleiben 154 g Reaktionsprodukt zurück, das 98,2 % Benzalhalogenid, 0,3 % Benzylhalogenid und 0,6 % Benzotrihalogenid enthält. Das Destillat, das 56,3 % Benzylhalogenid und 43,7 % Benzalhalogenid enthält, wird in die Halogenierung zurückgeführt. Im nächsten Cyclus beträgt die Ausbeute, bezogen auf neu eingesetztes t-Butyltoluol, ca. 98 % der Theorie an t-Butylbenzalhalogenid.

Beispiel 8

Apparatur und Ansatz wie in Beispiel 1 beschrieben.

Es werden jedoch 203,6 g (1,77 Mol) Bromchlorid eingesetzt. Nach 8 Stunden ist das eingesetzte Bromchlorid umgesetzt. Man erhält 240 g eines Gemisches, das 0,4% Benzylhalogenid, 84,1 % Benzalhalogenid und 14,3 % Benzotrihalogenid enthält. Dies entspricht einer Ausbeute an Benzalhalogeniden von 87 % der Theorie, bezogen auf eingesetzes Toluol.

Beispiel 9

Apparatur und Ansatz wie in Beispiel 1 beschrieben.

Es werden jedoch 277,2 g (2,40 Mol) Bromchlorid eingesetzt. Nach 10 Stunden ist die Reaktion beendet. Man erhält 281 g eines Gemisches, das 25 % Benzalhalogenid und 75% Benzotrihalogenid enthält.

Beispiel 10 (zum Vergleich)

4-tert.-Butyltoluol wird auf 170 bis 180°C aufgeheizt und unter Rühren und Bestrahlung mit einer Quecksilberhochdrucklampe wird Chlor eingeleitet. Es werden Proben genommen, deren Kernresonanzspektren zeigen, dass neben Benzyl- und Benzalchlorid in grösseren Mengen Chlor in die t-Butylgruppe eingeführt wird. Nachdem ca. 1,6 Äquivalente Chlor eingeleitet sind, zeigt das NMR-Spektrum, dass ein Gemisch vorliegt, das u.a. enthält:

4 Mol-% t-Butyltoluol
38 Mol-% t-Butylbenzylchlorid
14 Mol-% β-Chlor-t-butyltoluol
7 Mol-% t-Butylbenzalchlorid
2 Mol-% β,β-Dichlor-t-butyltoluol
5 Mol-% β,β'-Dichlor-t-butyltoluol
29 Mol-% β-Chlor-t-butylbenzylchlorid.

Chlorierungen mit Radikalstartern bei niedrigeren Temperaturen in Substanz oder Lösungsmittel führen zu ähnlichen Ergebnissen.

Die in den vorangegangenen Beispielen aufgeführten, mit Bromchlorid erhaltenen Gemische, geben in ihren NMR-Spektren keinen Hinweis auf β-halogenierte Produkte.

**Patentansprüche**

1. Verfahren zur Halogenierung von gegebenenfalls substituiertem 4-tert.-Butyltoluol, dadurch gekennzeichnet, dass man gegebenenfalls substituiertes 4-tert.-Butyltoluol mit 0,5 bis 3,2 Mol Bromchlorid, gegebenenfalls in Gegenwart von Chlor und/oder Brom, als Halogenierungsmittel, pro Mol gegebenenfalls substituiertes 4-tert.-Butyltoluol, bei Temperaturen von 40 bis 230°C, gegebenenfalls in Gegenwart von inerten organischen Lösungs- oder Verdünnungsmitteln, umsetzt.

2. Verfahren nach Anspruch 1, dadurch gekennzeichnet, dass man die Umsetzung mit 1,9 bis 2,2 Mol Halogenierungsmittel pro Mol Ausgangsprodukt durchführt.

3. Verfahren nach Anspruch 1, dadurch gekennzeichnet, dass man die Umsetzung mit 2,2 bis 3,2 Mol Halogenierungsmittel pro Mol Ausgangsprodukt durchführt.

4. Verfahren nach Anspruch 1, dadurch gekennzeichnet, dass man die Umsetzung mit 0,5 bis 1,9 Mol Halogenierungsmittel pro Mol Ausgangsprodukt durchführt.

5. Verfahren nach Ansprüchen 1 bis 4, dadurch gekennzeichnet, dass man die Umsetzung bei Temperaturen von 100 bis 200°C durchführt.

6. Gemisch enthaltend 90 bis 93 Gew.-% gegebenenfalls substituiertes 4-tert.-Butylbenzalhalogenid, 3 bis 4 Gew.-% gegebenenfalls substituiertes 4-tert.-Butylbenzylhalogenid und 4 bis 6 Gew.-% gegebenenfalls substituiertes 4-tert.-Butylbenzotrihalogenid, dadurch gekennzeichnet, dass das 4-tert.-Butylbenzalhalogenid 5 bis 30 Gew.-% eines Benzalhalogenids der Formel (II)

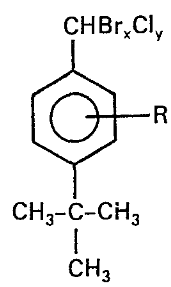

(II),

worin

X = 0 oder 1 und Y = 1 oder 2 sein kann, wobei die Summe von X + Y = 2 ist, und

R für Fluor, Chlor, Brom, Jod oder Wasserstoff steht,

und 70 bis 95 Gew.-% an gegebenenfalls substituiertem 4-tert.-Butylbenzalbromid enthält, das 4-tert.-Butylbenzylhalogenid 5 bis 30 Gew.-% an gegebenenfalls substituiertem 4-tert.-Butylbenzylchlorid und 70 bis 95 Gew.-% an gegebenenfalls substituiertem 4-tert.-Butylbenzylbromid enthält und das 4-tert.-Butylbenzotrihalogenid 5 bis 30 Gew.-% eines Benzotrihalogenids der Formel (III)

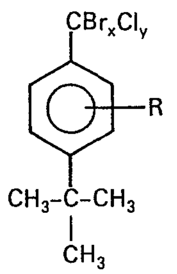

(III),

worin
X = 1 oder 2 und Y = 1 oder 2 sein kann, wobei die Summe von X + Y = 3 ist, und
R für Fluor, Chlor, Brom, Jod oder Wasserstoff steht
sowie 70 bis 95 Gew.-% an gegebenenfalls substituiertem 4-tert.-Butylbenzotribromid enthält.

7. Gemisch enthaltend 10 bis 95 Gew.-% gegebenenfalls substituiertes 4-tert.-Butylbenzotrihalogenid und 5 bis 90 Gew.-% gegebenenfalls substituiertes 4-tert.-Butylbenzalhalogenid, dadurch gekennzeichnet, dass das 4-tert.-Butylbenzalhalogenid 5 bis 30 Gew.-% eines Benzalhalogenids der Formel (II)

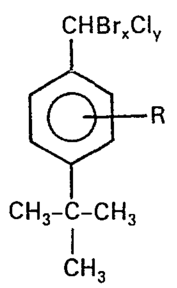

(II),

worin
X = 0 oder 1 und Y = 1 oder 2 sein kann, wobei die Summe von X + Y = 2 ist, und
R für Fluor, Chlor, Brom, Jod oder Wasserstoff steht, und 70 bis 95 Gew.-% an gegebenenfalls substituiertem 4-tert.-Butylbenzalbromid enthält, und das 4-tert.-Butylbenzotrihalogenid 5 bis 30 Gew.-% eines Benzotrihalogenids der Formel (III)

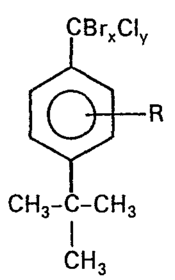

(III),

worin
X = 1 oder 2 und Y = 1 oder 2 sein kann, wobei die Summe von X + Y = 3 ist, und
R für Fluor, Chlor, Brom, Jod oder Wasserstoff steht
sowie 70 bis 95 Gew.-% an gegebenenfalls substituiertem 4-tert.-Butylbenzotribromid enthält.

8. Gemisch enthaltend 40 bis 85 Gew.-% gegebenenfalls substituiertes 4-tert.-Butylbenzalhalogenid und 15 bis 60 Gew.-% gegebenenfalls substituiertes 4-tert.-Butylbenzylhalogenid, dadurch gekennzeichnet, dass das 4-tert.-Butylbenzalhalogenid 5 bis 30 Gew.-% eines Benzalhalogenids der Formel (II)

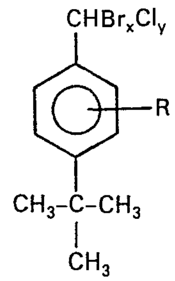

(II),

worin
X = 0 oder 1 und Y = 1 oder 2 sein kann, wobei die Summe von X + Y = 2 ist, und
R für Fluor, Chlor, Brom, Jod oder Wasserstoff steht, und 70 bis 95 Gew.-% an gegebenenfalls substituiertem 4-tert.-Butylbenzalbromid enthält, und das 4-tert.-Butylbenzylhalogenid 5 bis 30 Gew.-% an gegebenenfalls substituiertem 4-tert.-Butylbenzylchlorid und 70 bis 95 Gew.-% an gegebenenfalls substituiertem 4-tert.-Butylbenzylbromid enthält.

9. Gemisch enthaltend 95 bis 100 Gew.-% gegebenenfalls substituiertes 4-tert.-Butylbenzalhalogenid und 0 bis 5 Gew.-% gegebenenfalls substituiertes 4-tert.-Butylbenzylhalogenid, dadurch gekennzeichnet, dass das 4-tert.-Butylbenzalhalogenid 5 bis 30 Gew.-% eines Benzalhalogenids der Formel (II)

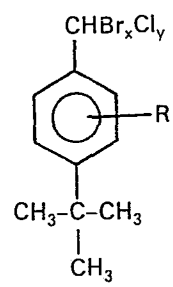

(II),

worin
X = 0 oder 1 und Y = 1 oder 2 sein kann, wobei die Summe von X + Y = 2 ist, und
R für Fluor, Chlor, Brom, Jod oder Wasserstoff steht,
und 70 bis 95 Gew.-% an gegebenenfalls substituiertem 4-tert.-Butylbenzalbromid enthält, und das 4-tert.-Butylbenzylhalogenid 5 bis 30 Gew.-% an gegebenenfalls substituiertem 4-tert.-Butylbenzylchlorid und 70 bis 95 Gew.-% an gegebenenfalls substituiertem 4-tert.-Butylbenzylbromid enthält.

**Revendications**

1. Procédé d'halogénation de 4-tertio-butyltoluène éventuellement substitué, caractérisé en ce qu'on fait réagir du 4-tertio-butyltoluène éventuellement substitué avec 0,5 à 3,0 moles de chlorure de brome, éventuellement en présence de chlore et/ou de brome, comme agent d'halogénation, pour chaque mole de 4-tertio-butyltoluène éventuellement substitué, à des températures de 40 à 230°C, éventuellement en présence de solvants ou de diluants organiques inertes.

2. Procédé suivant la revendication 1, caractérisé en ce qu'on conduit la réaction avec 1,9 à 2,2 moles d'agent d'halogénation par mole de matière de départ.

3. Procédé suivant la revendication 1, caractérisé en ce qu'on conduit la réaction avec 2,2 à 3,2 moles d'agent d'halogénation par mole de composant de départ.

4. Procédé suivant la revendication 1, caractérisé en ce qu'on conduit la réaction avec 0,5 à 1,9 mole d'agent d'halogénation par mole de composant de départ.

5. Procédé suivant les revendications 1 à 4, carctérisé en ce qu'on conduit la réaction à des températures de 100 à 200°C.

6. Mélange contenant 90 à 93 % en poids d'halogénure de 4-tertio-butylbenzal éventuellement substitué, 3 à 4 % en poids d'halogénure de 4-tertio-butylbenzyle éventuellement substitué et 4 à 6 % en poids d'halogénure de 4-tertio-butylbenzényle éventuellement substitué, caractérisé en ce que l'halogénure de 4-tertio-butylbenzal contient 5 à 30 % en poids d'un halogénure de benzal de formule (II)

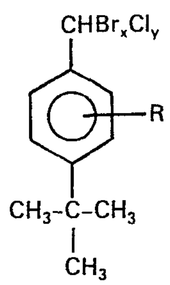

(II),

dans laquelle
X peut avoir la valeur 0 ou 1 et Y peut avoir la valeur 1 ou 2, la somme X + Y étant égale à 2, et
R désigne le fluor, le chlore, le brome, l'iode ou l'hydrogène,
et 70 à 95 % en poids de bromure de 4-tertio-butylbenzal éventuellement substitué, l'halogénure de 4-tertio-butylbenzyle contient 5 à 30 % en poids de chlorure de 4-tertio-butylbenzyle éventuellement substitué et 70 à 95 % en poids de bromure de 4-tertio-butylbenzyle éventuellement substitué et l'halogénure de 4-tertio-butylbenzényle contient 5 à 30 % en poids d'un halogénure de benzényle de formule (III)

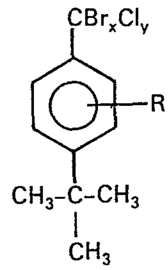

(III),

dans laquelle
X peut avoir la valeur 1 ou 2 et Y peut avoir la valeur 1 ou 2, la somme X + Y étant égale à 3, et
R désigne le fluor, le chlore, le brome, l'iode ou l'hydrogène,
ainsi que 70 à 95 % en poids de bromure de 4-tertio-butylbenzényle éventuellement substitué.

7. Mélange contenant 10 à 95 % en poids d'halogénure de 4-tertio-butylbenzényle éventuellement substitué et 5 à 90 % en poids d'halogénure de 4-tertio-butylbenzal éventuellement substitué, caractérisé en ce que l'halogénure de 4-tertio-butylbenzal contient 5 à 30 % en poids d'un halogénure de benzal de formule (II)

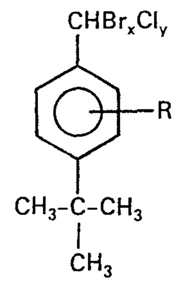

(II),

dans laquelle
X peut avoir la valeur 0 ou 1 et Y peut avoir la valeur 1 ou 2, la somme X + Y étant égale à 2, et
R désigne le fluor, le chlore, le brome, l'iode ou l'hydrogène, et 70 à 95 % en poids d'un bromure de 4-tertio-butylbenzal éventuellement substitué, et l'halogénure de 4-tertio-butylbenzényle contient 5 à 30 % en poids d'un halogénure de benzényle de formule (III)

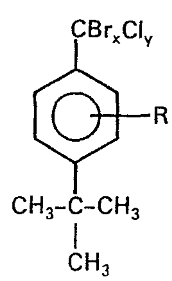

(III),

dans laquelle
X peut avoir la valeur 1 ou 2 et Y peut avoir la valeur 1 ou 2, la somme X + Y étant égale à 3, et
R désigne le fluor, le chlore, le brome, l'iode ou l'hydrogène,

ainsi que 70 à 95 % en poids de bromure de 4-tertio-butylbenzényle éventuellement substitué.

8. Mélange contenant 40 à 85 % en poids d'halogénure de 4-tertio-butylbenzal éventuellement substitué et 15 à 60 % en poids d'halogénure de 4-tertio-butylbenzyle éventuellement substitué, caractérisé en ce que l'halogénure de 4-tertio-butylbenzal contient 5 à 30 % en poids d'un halogénure de benzal de formule (II)

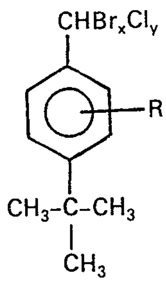

(II),

dans laquelle
X peut avoir la valeur 0 ou 1 et Y peut avoir la valeur 1 ou 2, la somme X + Y étant égale à 2, et
R désigne le fluor, le chlore, le brome, l'iode ou l'hydrogène, et 70 à 95 % en poids de bromure de 4-tertio-butylbenzal éventuellement substitué,
et l'halogénure de 4-tertio-butylbenzyle contient 5 à 30 % en poids de chlorure de 4-tertio-butylbenzyle éventuellement substitué et 70 à 95 % en poids de bromure de 4-tertio-butylbenzyle éventuellement substitué.

9. Mélange contenant 95 à 100 % en poids d'halogénure de 4-tertio-butylbenzal éventuellement substitué et 0 à 5 % en poids d'halogénure de 4-tertio-butylbenzyle éventuellement substitué, caractérisé en ce que l'halogénure de 4-tertio-butylbenzal contient 5 à 30 % en poids d'un halogénure de benzal de formule (II)

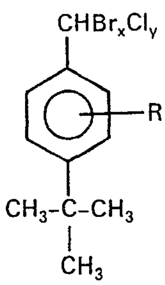

(II),

dans laquelle
X peut avoir la valeur 0 ou 1 et Y peut avoir la valeur 1 ou 2, la somme X + Y étant égale à 2, et
R désigne le fluor, le chlore, le brome, l'iode ou l'hydrogène, et 70 à 95 % en poids de bromure de 4-tertio-butylbenzal éventuellement substitué, et l'halogénure de 4-tertio-butylbenzyle contient 5 à 30 % en poids de chlorure de 4-tertio-butyle éventuellement substitué et 70 à 95 % en poids de bromure de 4-tertio-butylbenzyle éventuellement substitué.

**Claims**

1. Process for the halogenation of optionally substituted 4-tert.-butyltoluene, characterised in that optionally substituted 4-tert.-butyltoluene is reacted with 0.5 to 3.2 mols of bromine chloride, if appropriate in the presence of chlorine and/or bromine, as the halogenating agent, per mol of optionally substituted 4-tert.-butyltoluene, at temperatures of 40 to 230°C and if appropriate in the presence of inert organic solvents or diluents.

2. Process according to Claim 1, characterised in that the reaction is carried out with 1.9 to 2.2 mols of halogenating agent per mol of starting material.

3. Process according to Claim 1, characterised in that the reaction is carried out with 2.2 to 3.2 mols of halogenating agent per mol of starting material.

4. Process according to Claim 1, characterised in that the reaction is carried out with 0.5 to 1.9 mols of halogenating agent per mol of starting material.

5. Process according to Claims 1 to 4, characterised in that the reaction is carried out at temperatures of 100 to 200°C.

6. Mixture containing 90 to 93% by weight of optionally substituted 4-tert.-butylbenzal halide, 3 to 4% by weight of optionally substituted 4-tert.-butylbenzyl halide and 4 to 6% by weight of optionally substituted 4-tert.-butylbenzotrihalide, characterised in that the 4-tert.-butylbenzal halide contains 5 to 30% by weight of a benzal halide of the formula (II)

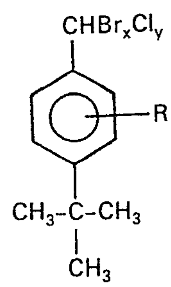

(II),

wherein
X can be 0 or 1 and
Y can be 1 or 2, the total of X + Y being 2, and
R represents fluorine, chlorine, bromine, iodine or hydrogen,
and 70 to 95% by weight of optionally substituted 4-tert.-butylbenzal bromide, the 4-tert.-butylbenzyl halide contains 50 to 30% by weight of optionally substituted 4-tert.-butylbenzyl chloride and 70 to 95% by weight of optionally substituted 4-tert.-butylbenzyl bromide, and the 4-tert.-butylbenzotrihalide contains 5 to 30% by weight of a benzotrihalide of the formula (III)

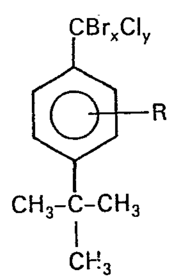

(III),

wherein
X can be 1 or 2 and
Y can be 1 or 2, the total of X + Y being 3, and
R represents fluorine, chlorine, bromine, iodine or hydrogen,
and 70 to 95% by weight of optionally substituted 4-tert.-butylbenzotribromide.

7. Mixture containing 10 to 95% by weight of optionally substituted 4-tert.-butylbenzotrihalide and 5 to 90% by weight of optionally substituted 4-tert.-butylbenzal halide, characterised in that the 4-tert.-butylbenzal halide contains 5 to 30% by weight of a benzal halide of the formula (II)

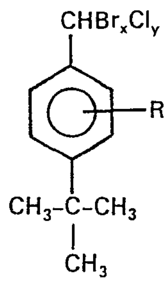

(II),

wherein
X can be 0 or 1 and
Y can be 1 or 2, the total of X + Y being 2, and
R represents fluorine, chlorine, bromine, iodine or hydrogen,
and 70 to 90% by weight of optionally substituted 4-tert.-butylbenzal bromide, and the 4-tert.-butylbenzotrihalide contains 5 to 30% by weight of a benzotrihalide of the formula (III)

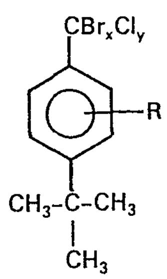

(III),

wherein
X can be 1 or 2 and
Y can be 1 or 2, the total of X + Y being 3, and
R represents fluorine, chlorine, bromine, iodine or hydrogen,
and 70 to 95% by weight of optionally substituted 4-tert.-butylbenzotribromide.

8. Mixture containing 40 to 85% by weight of optionally substituted 4-tert.-butylbenzal halide and 15 to 60% by weight of optionally substituted 4-tert.-butylbenzyl halide, characterised in that the 4-tert.-butylbenzal halide contains 5 to 30% by weight of a benzal halide of the formula (II)

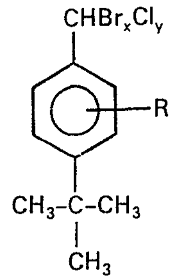

(II),

wherein
X can be 0 or 1 and
Y can be 1 or 2, the total of X + Y being 2, and
R represents fluorine, chlorine, bromine, iodine or hydrogen,
and 70 to 95% by weight of optionally substituted 4-tert.-butylbenzal bromide, and the 4-tert.-butylbenzyl halide contains 5 to 30% by weight of optionally substituted 4-tert.-butylbenzyl chloride and 70 to 95% by weight of optionally substituted 4-tert.-butylbenzyl bromide.

9. Mixture containing 95 to 100% by weight of optionally substituted 4-tert.-butylbenzal halide and 0 to 5% by weight of optionally substituted 4-tert.-butylbenzyl halide, characterised in that the 4-tert.-butylbenzal halide contains 5 to 30% by weight of a benzal halide of the formula (II)

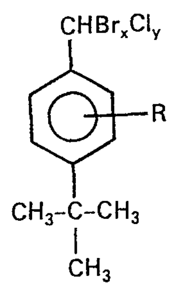

(II),

wherein
X can be 0 or 1 and
Y can be 1 or 2, the total of X + Y being 2, and
R represents fluorine, chlorine, bromine, iodine or hydrogen,
and 70 to 95% by weight of optionally substituted 4-tert.-butylbenzal bromide, and the 4-tert.-butylbenzyl halide contains 5 to 30% by weight of optionally substituted 4-tert.-butylbenzyl chloride and 70 to 95% by weight of optionally substituted 4-tert.-butylbenzyl bromide.